# EUROPÄISCHE PATENTSCHRIFT

(11) **EP 4 181 837 B1**
(45) Veröffentlichungstag und Bekanntmachung des Hinweises auf die Patenterteilung: **05.11.2025**
(21) Anmeldenummer: 21754713.2
(22) Anmeldetag: 20.07.2021
(51) Int. Cl.: A61F 2/60, A61F 2/64, A61F 2/68, A61F 2/50, A61F 2/76

(54) **VERFAHREN ZUR STEUERUNG EINER PROTHESE ODER ORTHESE**
METHOD FOR CONTROLLING A PROSTHESIS OR ORTHESIS
PROCÉDÉ DE COMMANDE D'UNE PROTHÈSE OU ORTHÈSE

(30) Priorität: 20.07.2020 DE 102020004338
(43) Veröffentlichungstag der Anmeldung: 24.05.2023
(73) Patentinhaber: Otto Bock Healthcare Products GmbH, 1110 Wien (AT)
(72) Erfinder: HOFMANN, Thomas, 1030 Wien (AT); SEIFERT, Dirk, 1070 Wien (AT)
(74) Vertreter: Gramm, Lins & Partner Patent- und Rechtsanwälte PartGmbB
(86) Internationale Anmeldenummer: PCT/EP2021/070284
(87) Internationale Veröffentlichungsnummer: WO 2022/018092

(56) Entgegenhaltungen:
- DE-A1- 102015 106 384
- DE-T2- 60 131 377

## Beschreibung

Die Erfindung betrifft ein Verfahren zur Steuerung einer Prothese oder Orthese der unteren Extremität gemäß Anspruch 1.

Künstliche Kniegelenke werden in Prothesen und Orthesen sowie in Exoskeletten als Sonderfall von Orthesen verwendet. Ein künstliches Kniegelenk weist ein Oberteil und ein Unterteil auf, die relativ zueinander verschwenkbar um eine Gelenkachse, die Knieachse, gelagert sind. Im einfachsten Fall ist das Kniegelenk als ein Einachskniegelenk ausgebildet, bei dem beispielsweise ein Bolzen oder zwei auf einer Schwenkachse angeordnete Lagerstellen eine einzelne Knieachse bilden. Ebenfalls sind künstliche Kniegelenke bekannt, die keine festgelegte Drehachse zwischen Oberteil und Unterteil ausbilden, sondern entweder abgleitende oder abrollende Oberflächen oder eine Vielzahl von gelenkig miteinander verbundenen Lenkern aufweisen. Um die Bewegungseigenschaften der Kniegelenke beeinflussen zu können und ein an das natürliche Gangverhalten angenähertes Bewegungsverhalten der Orthese oder Prothese bzw. dem Exoskelett zu erhalten, sind zwischen dem Oberteil und dem Unterteil Widerstandseinrichtungen vorgesehen, über die der jeweilige Widerstand verändert werden kann. Rein passive Widerstandseinrichtungen sind passive Dämpfer, beispielsweise Hydraulikdämpfer, Pneumatikdämpfer oder Dämpfer, die auf Grundlage magnetorheologischer Effekte den Bewegungswiderstand verändern. Ebenfalls existieren aktive Widerstandseinrichtungen, beispielsweise Motoren oder andere Antriebe, die über eine entsprechende Verschaltung als Generator oder Energiespeicher betreibbar sind.

Die jeweiligen Kniegelenke, also die Prothesengelenke oder Orthesenkniegelenke, werden mit jeweiligen Anschlussmitteln an den Patienten festgelegt. Bei Prothesenkniegelenken erfolgt die Festlegung in der Regel über einen Oberschenkelschaft, der einen Gliedmaßenstumpf aufnimmt. Alternative Festlegungsarten sind ebenfalls möglich, beispielsweise durch osseointegrierte Anschlussmittel oder über Gurte und andere Einrichtungen. Bei Orthesen und Exoskeletten werden das Oberteil und Unterteil unmittelbar an dem Oberschenkel und dem Unterschenkel festgelegt. Die dafür vorgesehenen Befestigungseinrichtungen sind beispielsweise Gurte, Manschetten, Schalen oder Rahmenkonstruktionen. Orthesen können auch Fußteile zum Aufsetzen eines Fußes oder Schuhes aufweisen. Die Fußteile können gelenkig an dem Unterteil gelagert sein.

Ein künstliches Kniegelenk weist in der konstruktiv maximal erreichbaren Streckung einen Kniewinkel von 180° auf, eine Hyperextension, also ein Winkel auf der posterioren Seite von mehr als 180° ist in der Regel nicht vorgesehen. Das Verschwenken des Unterteils gegenüber dem Oberteil nach posterior wird als Knie-Flexion bezeichnet, ein Verschwenken nach anterior oder nach vorn in die Richtung als Extension.

Aus der EP 2 498 729 B1 ist ein Verfahren zur Steuerung eines künstlichen orthetischen oder prothetischen Gelenkes einer unteren Extremität mit einer Widerstandseinrichtung bekannt, der zumindest ein Aktuator zugeordnet ist, über den der Beuge- und/oder Streckwiderstand in Abhängigkeit von Sensordaten verändert wird, wobei während der Benutzung des Gelenkes über Sensoren Zustandsinformationen bereitgestellt werden. Wird eine vom Gehen abweichende zyklische Bewegung ermittelt, wird der Widerstand für die Dauer der zyklischen Bewegung reduziert. Um die zyklische Bewegung zu ermitteln, werden die zeitlichen Abstände zwischen den Maxima und den Minima des Kniewinkels und die charakteristischen Kniewinkelverkäufe herangezogen. Werden keine Maxima und Minima erkannt, wird der Widerstand erhöht.

Aus der DE 601 31 377 T2 ist ein Verfahren zur angepassten Steuerung einer Knieprothese bekannt, bei dem in einem Speicher Korrelationen zwischen sensorischen Daten und der Dämpfung der Schwungphase gespeichert werden. Die Korrelationen werden in klinischen Untersuchungen von Patienten mit Amputationen mit unterschiedlichen Körpergrößen ermittelt. Sensoren an der Knieprothese nehmen während der Nutzung der Prothese sensorische Daten auf, die mit der Schwungphasendämpfung korreliert werden, um die Schwungphasendämpfung automatisch einzustellen, ohne dass eine spezielle Patienteninformation vorher in der Knieprothese einprogrammiert wird.

Aus der DE 10 2015 106 384 A1 betrifft ein Verfahren zur Steuerung einer Widerstandsänderung bei einem künstlichen Gelenk, das ein Oberteil und ein Unterteil aufweist, die um eine Schwenkachse verschwenkbar aneinander befestigt sind. Eine Widerstandseinrichtung ist zwischen dem Oberteil und dem Unterteil befestigt, um einen Widerstand gegen eine Einbeugung oder Streckung des künstlichen Gelenkes bereitzustellen. Über eine der Widerstandseinrichtungen zugeordnete Verstelleinrichtung wird der Widerstand verändert, wenn eine Steuerungseinrichtung aufgrund eines Sensorsignals die Verstelleinrichtung aktiviert. Der Widerstand wird dabei in Abhängigkeit von der Lage und/oder der Länge einer gemessenen oder berechneten Beinsehne bzw. deren zeitlicher Ableitungen verändert. Als Beinsehne wird insbesondere die Verbindungslinie zwischen einem Drehpunkt und einem Fußpunkt verwendet.

Problematisch ist hierbei, dass beim Fahrradfahren Situationen auftreten können, in denen nicht gleichmäßig getreten wird. Beispielsweise bei Bergabfahrten oder wenn der Nutzer aufstehen muss, um über ein Hindernis zu fahren.

Aufgabe der vorliegende Erfindung ist es, ein Verfahren bereitstellen, mit dem das Fahrradfahren mit einem künstlichen Kniegelenk vereinfacht wird.

Erfindungsgemäß wird diese Aufgabe durch ein Verfahren mit den Merkmalen des Hauptanspruches gelöst. Vorteilhafte Ausgestaltungen und Weiterbildungen der Erfindung sind in den Unteransprüchen, der Beschreibung und den Figuren offenbart.

Das Verfahren zur Steuerung einer Prothese oder Orthese der unteren Extremität mit einem Oberteil und einem mit dem Oberteil über ein Kniegelenk verbundenes Unterteil, das um eine Gelenkachse relativ zu dem Oberteil verschwenkbar gelagert ist, wobei zwischen dem Oberteil und dem Unterteil eine verstellbare Widerstandseinrichtung angeordnet ist, über die auf der Grundlage von Sensordaten ein Widerstand verändert wird, wobei Zustandsinformationen über Sensoren erfasst, eine vom Gehen abweichende zyklische Bewegung ermittelt und der Widerstand während der zyklischen Bewegung auf ein niedriges Niveau eingestellt wird, sieht vor, dass die Ermittlung der zyklischen Bewegung die Erfassung des Flexionswinkels und zumindest eines Absolutwinkels des Unterteils und/oder des Oberteils über zumindest einen Bewegungszyklus und die Erkennung der zyklischen Bewegung aus der Relativbewegung von Oberteil und Unterteil und der Absolutbewegungen von Oberteil und/oder Unterteil im Raum aufweist, wobei zur Ermittlung der zyklischen Bewegung der Phasenraum zweier Winkel-Kenngrößen und/oder deren Ableitungen herangezogen wird, insbesondere die Drehrichtung im Phasenraum bestimmt und herangezogen wird. Der Flexionswinkel muss dabei nicht direkt über einen Winkelsensor gemessen werden, er kann auch über die Absolutwinkel des Oberteils und des Unterteils errechnet werden. Über die Erfassung des Flexionswinkels, also der Relativverschwenkung zwischen dem Oberteil und dem Unterteil ausgehend von einer gestreckten Stellung mit einem Kniewinkel von 180°, und der Erfassung zumindest eines Absolutwinkels des Oberteils bzw. des Unterteils über zumindest einen Inertialwinkelsensor oder eine IMU ist es möglich, die Relativbewegung von Oberteil und Unterteil zueinander sowie die Bewegung im Raum zu erkennen. Die dauerhafte Erfassung sowohl des Flexionswinkels als auch eines oder mehrerer Absolutwinkel, also des Absolutwinkels des Oberteils und/oder des Absolutwinkels des Unterteils, ermöglicht ein einfaches Umschalten in die Fahrradfunktion des künstlichen Kniegelenkes, ohne dass eine zusätzliche Aktivität durch den Orthesennutzer oder Prothesennutzer ausgeführt werden muss. Es muss keine Schalterstellung geändert, keine App aktiviert oder ein besonderes Bewegungsmuster durchgeführt werden, vielmehr wird allein durch die Pedalierbewegung das Umschalten in die Fahrradfunktion ausgelöst. Die Fahrradfunktion wird solange beibehalten, bis zumindest ein Beendigungskriterium detektiert wird. Eine fortgesetzte, ununterbrochene zyklische Bewegung, die insbesondere vom Gehen abweicht, muss nicht vorliegen. Wird z.B. beim Absteigen die Prothese oder die Orthese auf dem Boden aufgesetzt, wird die Fahrradfunktion deaktiviert, während des ununterbrochenen Fahrens, auch ohne zyklische Bewegungen, beispielsweise wenn der Nutzer stehend in den Pedalen fährt, wird die Funktion vorteilhafterweise nicht deaktiviert, sodass nicht erneut ein vollständiger Erkennungszyklus durchgelaufen werden muss, um eine Fahrradfunktion einzuschalten. Für die Fahrradfunktion werden nach dem Erkennen der zyklischen Bewegung vorteilhafterweise der Extensionswiderstand und der Flexionswiderstand reduziert. Wird nachfolgend von dem Widerstand allgemein gesprochen, sind Flexionswiderstand und Extensionswiderstand einzeln oder gemeinsam genannt, wird ausdrücklich von Extensionswiderstand oder Flexionswiderstand gesprochen, ist nur dieser Widerstand gemeint. Zur Ermittlung der zyklischen Bewegung wird der Phasenraum zweier Winkel-kenngrößen und oder deren Ableitungen herangezogen, insbesondere wird die Drehrichtung im Phasenraum bestimmt und herangezogen, um zu erkennen, ob eine zyklische Bewegung abweichend vom Gehen in der Ebene vorliegt und eine entsprechende Reduzierung des Widerstandes ausgeführt wird. Die Winkelkenngrößen sind der Flexionswinkel und zumindest ein Absolutwinkel einer der Komponenten um das Kniegelenk herum. Es werden die Winkel im Raum für das Unterteil und/oder das Oberteil verwendet.

Eine Weiterbildung der Erfindung sieht vor, dass die Reduzierung des Widerstandes nur dann erfolgt, wenn eine Flexionswinkeländerung, insbesondere der Betrag der Flexionswinkeländerung, aufsummiert über ein Intervall, in dem bestimmte Bedingungen erfüllt sind, größer als ein festgelegter Grenzwert, insbesondere größer als 240° ist. Um eine zyklische Pedalierbewegung zu erkennen und dadurch die Fahrradfunktion zu aktivieren, ist es vorteilhaft, wenn sowohl der Flexionswinkel als auch die Absolutwinkel des Oberteils und/oder des Unterteils bestimmte Kriterien erfüllen, und zwar über einen bestimmten Zeitraum, damit der Einschaltbefehl erfolgt. Vorteilhafterweise wird dieser Zeitraum über den die Flexionswinkeländerung bestimmt. Die Aufsummierung des Betrages des Flexionswinkels hat den Vorteil, dass die Erfassung der Kenngröße Flexionswinkel kontinuierlich erfolgen kann und keine Unterscheidung zwischen Flexion und Extension getroffen werden muss. Würde der Flexionswinkel mit Vorzeichen aufsummiert, wäre nach einer Pedalumdrehung die Summe Null und eine zyklische Bewegung könnte nie festgestellt werden, da diese eine Wiederholung benötigt. Alternativ erfolgt die Aufsummierung der Flexionswinkeländerung nur in bestimmten Zeitintervallen oder bei bestimmten Bedingungen, z.B. nur bi zunehmenden Flexionswinkel oder nur bei abnehmenden Flexionswinkel oder bei einer Veränderung nur unter einer bestimmten Axialbelastung des Unterteils oder bei gleichzeitiger Winkelveränderung eines Fußteiles zu dem Unterteil. Der Flexionswinkel wird überwacht und dessen Änderung wird in bestimmten Zeitintervallen kumuliert oder aufsummiert. Liegen über den Zeitraum der kumulierten absoluten Flexionswinkeländerungen die notwendigen Bedingungen für die Erkennung des zyklischen Bewegung und der Aktivierung des Fahrradfahrmodus vor, wird der Widerstand reduziert.

Ist eine Bedingung über den Zeitraum der Ermittlung der zyklischen Bewegung nicht mehr erfüllt, liegt beispielsweise der Flexionswinkel nicht innerhalb der vorgegebenen Grenzwerte oder wird das Unterteil und/oder das Oberteil so verlagert, dass sie nicht innerhalb der vorgegebenen Parameter befindlich sind, wird der Zähler für den Flexionswinkel wieder zurückgesetzt. Dadurch wird verhindert, dass zufällige Bewegungen oder Übereinstimmungen mit den Parametern über den Tag verteilt aufsummiert werden. Die Grenzwerte oder Parameter müssen über den festgelegten Zeitraum, beispielsweise die Summe der Flexionswinkeländerung oder die kumulierte, absolute Flexionswinkeländerung erfüllt sein. Bei einer Unterbrechung des Vorliegens der notwendigen Bedingungen vor Aktivierung des Fahrradmodus wird keine Verringerung des Widerstandes veranlasst und nicht in den Fahrtmodus geschaltet.

Bevorzugt ist vorgesehen, dass die Reduzierung des Widerstandes nur dann erfolgt, wenn die aufgestellten Bedingungen für den Flexionswinkel und den Neigungswinkel des Unterteils über die kumulierte Flexionswinkeländerung, z.B. die Summe des Betrages der Flexionswinkeländerung größer als 240°, vorliegen oder erfüllt sind. Die notwendigen Bedingungen für eine Widerstandsverringerung müssen über den gesamten Zeitraum der kumulierten Flexionswinkeländerung, z.B. größer als 240° vorliegen, ansonsten wird die Fahrradfunktion nicht aktiviert. Ist die Aktivierung der Fahrradfunktion durchgeführt, liegen also die notwendigen Bedingungen über einen festgelegten Zeitraum einer Flexionswinkeländerung, insbesondere größer als 240° vor, ist es nicht mehr notwendig, die gleichförmige, zyklische Bewegung auszuführen, um weiterhin den reduzierten Widerstand vorliegen zu haben. Der reduzierte Widerstand wird beibehalten, solange bis sich definierte Änderungen ergeben oder Sensorwerte detektiert werden, die eine Vergrößerung des Flexionswiderstandes und/oder Extensionswiderstandes notwendig machen.

Eine Weiterbildung der Erfindung sieht vor, dass eine zyklische Bewegung nur dann festgestellt wird, wenn der Flexionswinkel größer als ein festgelegter Grenzwert, insbesondere größer 10°, insbesondere größer als 15° ist. Der Flexionswinkel größer als 10°, insbesondere größer als 15°, ist ein guter Indikator dafür, dass eine Pedalierbewegung durchgeführt wird. Beim Fahrradfahren, zumindest im Sitzen, ist oder sollte zu Beginn ein Flexionswinkel stets größer 10°, insbesondere größer als 15° vorhanden sein. Ist dies der Fall, kann mit hoher Sicherheit davon ausgegangen werden, dass eine Pedalierbewegung vorliegt.

Eine Weiterbildung der Erfindung sieht vor, dass eine zyklische Bewegung nur dann festgestellt wird, wenn der Neigungswinkel des Unterteils und/oder der Flexionswinkel bestimmte Grenzwerte nicht über- und/oder unterschreiten. Der Neigungswinkel des Unterteils im Raum und dessen Verlauf über die Zeit ist ein guter Indikator dafür, ob eine zyklische Bewegung vorliegt oder nicht und ob die zyklische Bewegung aufrechterhalten wird oder nicht. In Kombination mit dem Flexionswinkel können diejenigen Parameter eingegrenzt werden, die für das Vorliegen oder Nichtvorliegen einer zyklischen Bewegung aussagekräftig sind. Wenn der Neigungswinkel des Unterteils über einen festgelegten Zeitraum innerhalb bestimmter Grenzwerte liegt, also die Grenzwerte nicht überschreitet oder unterschreitet und gleiches auch für den Flexionswinkel gilt, kann daraus auf das Vorliegen oder Nichtvorliegen der zyklischen Bewegung geschlossen werden. Wird das Vorliegen der zyklischen Bewegung detektiert, wird der Widerstand verringert, wenn der Fahrradmodus nicht eingeschaltet oder aktiviert werden soll, bleibt der Widerstand auf dem Ausgangsniveau, beispielsweise dem Standphasenniveau für das Gehen in der Ebene.

Eine Weiterbildung der Erfindung sieht vor, dass eine zyklische Bewegung nur dann festgestellt wird, wenn der Neigungswinkel des Unterteils relativ zu der Senkrechten einen Grenzwert nicht überschreitet, insbesondere kleiner als 5° ist. Der Neigungswinkel des Unterteils, der sogenannte Rollwinkel, ist ebenfalls ein Indikator dafür, dass eine Fahrradfahrtätigkeit ausgeführt wird. Wenn der Neigungswinkel des Unterteils nach vorn relativ zu der Senkrechten über eine bestimmte Zeit stets kleiner als ein Grenzwert ist, insbesondere kleiner als 5°, wobei eine weiter nach vorne gerichtete Neigung unschädlich ist, kann davon ausgegangen werden, dass es sich hierbei um Fahrradfahrbewegung handelt. Die Neigung des Unterteils nach hinten, wenn das Fußteil oder die Knöchelgelenksachse sich vor der Kniegelenksachse befindet, wird als positiver Rollwinkel angenommen. Eine geringe Neigung nach hinten kann toleriert werden und kommt beim Fahrradfahren vor, der Grenzwert sollte nicht überschritten werden, insbesondere nicht größer als 5° betragen.

Eine Weiterbildung der Erfindung sieht vor, dass die Beendigung der zyklischen Bewegung dadurch erkannt wird, dass das Unterteil für einen vorgegebenen Zeitraum, insbesondere größer als >150ms, von einer Axiallast oder Axialkraft entlastet wird und der Flexionswinkel einen Grenzwert, insbesondere kleiner als 15°, unterschreitet oder die Neigung des Unterteils zur Senkrechten einen Grenzwert, insbesondere größer als 10°, überschreitet. Entlastung des Unterteils von einer Axialkraft kann darin bestehen, dass die Axialkraft, die in Richtung auf das Kniegelenk wirkt, unter einen vorgegebenen Grenzwert sinkt oder eine vollständige Entlastung des Unterteils vorhanden ist, also keine Axialkraft in Richtung auf das Kniegelenk wirkt . Wird für einen vorgegebenen Zeitraum das Unterteil entlastet und wirkt keine in Richtung der Gelenkachse wirkende Axialkraft auf das Unterteil, kann dies als ein Anhaltswert dafür gelten, dass der Nutzer den Fuß von der Pedale genommen hat und somit ggf. absteigen will oder aber den Fuß absetzt oder aber den Fuß absetzen muss, um sich anderweitig abzustützen. Unterschreitet der Flexionswinkel darüber hinaus einen Grenzwert, insbesondere kleiner als 15°, sodass eine Streckung des versorgten Beines angenommen werden kann, oder überschreitet die Neigung des Unterteils zur Senkrechten einen Grenzwert, insbesondere größer als 10°, was beispielsweise beim Absteigen und Überschwingen des Beines über das Hinterrad und den Sattel erfolgt, wird vorteilhafterweise das Fahrradfahren als beendet erkannt und insbesondere der Flexionswiderstand wieder erhöht. Die zyklische Bewegung muss nicht dauerhaft von Beginn an ausgeführt werden, lediglich über einen gewissen Zeitraum, nämlich über eine gewisse Anzahl an Pedalumdrehungen oder über eine gewisse Summe einer Flexionswinkeländerung, um den reduzierten Widerstand einzustellen.

Eine Weiterbildung der Erfindung sieht vor, dass bei Unterbrechung der zyklischen Bewegung bei gleichzeitiger Beaufschlagung des Unterteils mit einer Axialkraft eine Extension des Kniegelenkes unterhalb eines festgelegten Flexionswinkels, insbesondere unterhalb 10°, verhindert und der Flexionswiderstand erhöht wird und nach Erreichen eines Endanschlages, einer nachfolgender Flexion und erneuter Extension die Widerstandseinstellung für die zyklische Bewegung erneut eingestellt wird. Wird beispielsweise eine Kniestreckung detektiert, was eine Unterbrechung der zyklischen Bewegung, nicht jedoch eine Unterbrechung des Fahrradfahrens und damit eine Unterbrechung des eingestellten Widerstandsmodus in der Widerstandseinrichtung darstellt und gleichzeitig eine Axialkraft detektiert, wird eine Extension des Kniegelenkes über einen bestimmten Wert hinaus verhindert, beispielsweise über einen Extensionswert kleiner als 10°, um eine vollständige Streckung des Kniegelenkes zu verhindern. Eine vollständige Streckung bei einer Unterbrechung der Tretbewegung, beispielsweise beim Aufstehen, kann zu einer erschwerten Beugung des Kniegelenkes führen, was nicht erwünscht ist, wenn die Pedalierbewegung wieder aufgenommen werden soll. Gleichzeitig wird der Flexionswiderstand erhöht, beispielsweise auf ein Standphasenniveau beim Gehen, was eine grundsätzliche Flexion ermöglicht. Dadurch wird auch für ein Hinsetzen nach dem Aufstehen eine ausreichend hohe Flexionsdämpfung oder ein Flexionswiderstand bereitgestellt, um eine kontrollierte Kniebeugung zu ermöglichen. Wird der Endanschlag der Extension erreicht und findet eine nachfolgende Flexion gegen den dann ggf. erhöhten Widerstand statt, wird bei einer Bewegungsumkehr und erneuten Extension die Widerstandseinstellung für die zyklische Bewegung erneut eingestellt, also der Flexionswiderstand und ggf. der Extensionswiderstand reduziert. Dadurch wird verhindert, dass die Gleichförmigkeitskriterien hinsichtlich des Flexionswinkels und des Neigungswinkels des Unterteils wieder über den vollständigen notwendigen Wert der kumulierten, absoluten Flexionswinkeländerung, beispielsweise den Betrag der Flexionswinkeländerung, vorliegen müssen. Beispielsweise wird so die Situation des Aufstehens, beispielsweise beim Überfahren eines Hindernisses, erkannt und eine vereinfachte Freischaltung der Widerstandseinrichtung ermöglicht.

Eine Weiterbildung der Erfindung sieht vor, dass zur Ermittlung der zyklischen Bewegung die Winkelgeschwindigkeiten errechnet und der Quotient der Winkelgeschwindigkeiten in festgelegten Zeitabschnitten ermittelt wird. Änderungen des Quotienten der Winkelgeschwindigkeiten werden als Tangentensteigungen in festgelegten Zeitabschnitten ermittelt. Eine Feststellung der zyklischen Bewegung erfolgt, wenn der zeitliche Verlauf der Tangentensteigungen monoton steigend ist. Ein monoton steigender zeitlicher Verlauf der Tangentensteigung liegt auch dann vor, wenn kleinere Abweichungen der Monotoniebedingung vorhanden sind. Wesentlich ist, dass die Änderung der Steigung dabei nicht unter einen festgelegten Grenzwert fällt. Die Errechnung der Winkelgeschwindigkeiten, also der Winkelgeschwindigkeiten des Flexionswinkels und des Neigungswinkels des Unterteils sowie die Errechnung des Quotientens der Winkelgeschwindigkeiten in festgelegten Zeitabschnitten ermöglicht es, dass Tangentensteigungen des Verlaufs der beiden Winkelgeschwindigkeiten, die als geschlossene, konvexe zweidimensionale Kurve dargestellt werden kann, ermittelt werden können. Die Feststellung der zyklischen Bewegung und damit einer Verringerung des Widerstandes ist dann anzunehmen, wenn über einen vollständigen Umlauf der Kurve die Tangentensteigung als monoton steigend festgestellt wird.

Eine Weiterbildung der Erfindung sieht vor, dass eine Auswertung der Winkelgeschwindigkeiten nur bei Überschreiten eines Grenzwertes der Flexionswinkelgeschwindigkeit erfolgt. Die Berücksichtigung der Flexionswinkelgeschwindigkeit gibt einen zusätzlichen Anhaltspunkt darauf, dass tatsächlich eine Fahrradfahrbewegung stattfindet und nicht zufällig kleine widerkehrende Bewegungen, beispielsweise beim Sitzen, irrtümlich als Fahrradfahrbewegung erkannt werden. Darüber hinaus wird bei der Bestimmung der Tangentensteigung dadurch die Betrachtung von Unstetigkeitsstellen vermieden.

Eine Weiterbildung der Erfindung sieht vor, dass aus dem Kniewinkel und/oder den Absolutwinkel von Oberteil und Unterteil die Bahnkurve des Fußteils und oder deren Ableitungen relativ zu einem ermittelten Hüftdrehpunkt bestimmt wird oder werden und für die Ermittlung der zyklischen Bewegung herangezogen wird. Aus dem Kniewinkel, den Absolutwinkeln oder einem Absolutwinkel sowie den bekannten geometrischen Beziehungen zwischen der Knieachse und dem Fußteil, beispielsweise einem Referenzpunkt des Fußteils in Fußsohlennähe oder die Position der Knöchelgelenksachse ist es möglich, die Bahnkurve des Fußteils zu bestimmen. Die Bahnkurve oder deren zeitliche Ableitungen können zu einem ermittelten Drehpunkt in Relation gesetzt werden. Der Hüftdrehpunkt wird von einem Orthopädietechniker während der Anpassung der Orthese oder Prothese ohnehin bestimmt. Der Abstand zu der Kniegelenksachse ist daher ebenfalls bekannt. Aus den jeweils bekannten geometrischen Beziehungen und Winkeländerungen können dann der Bahnkurvenverlauf, die Bahnkurvengeschwindigkeiten und/oder Bahnkurvenbeschleunigungen eines Referenzpunktes eines Fußteils zu dem Drehpunkt ermittelt werden und als Indikator dafür dienen, ob eine zyklische Bewegung vorliegt oder nicht.

Nachfolgend wird ein Ausführungsbeispiel der Erfindung anhand der Figuren erläutert. Es zeigen:
- Figur 1 -: eine schematische Darstellung eines Prothesenbeines;
- Figur 2 -: eine schematische Darstellung einer Pedalierbewegung;
- Figur 3 -: eine gemeinsame Darstellung eines Flexionswinkels und eines Rollwinkels;
- Figur 4 -: eine schematische Darstellung der Berechnung einer Tangentensteigung;
- Figur 5 -: eine Gegenüberstellung und gemessenen und errechneten Winkelgeschwindigkeiten;
- Figur 6 -: den Verlauf der Steigung einer Verbindungslinie in dem Diagramm gemäß Figur 5;
- Figur 7 -: eine Datenaufzeichnung für das Losfahren und die Aktivierung einer Fahrradfahrfunktion; sowie
- Figur 8 -: eine Darstellung einer Orthese.

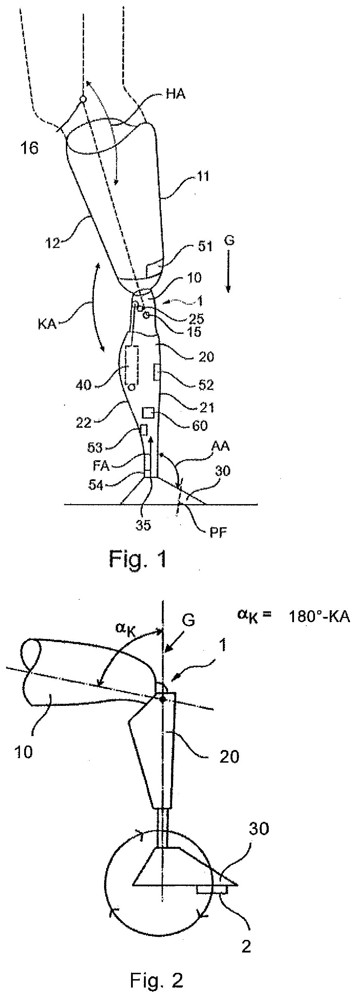

Figur 1 zeigt eine schematische Darstellung eines künstlichen Kniegelenkes 1 in einer Anwendung an einem Prothesenbein. Alternativ zu einer Anwendung an einem Prothesenbein kann ein entsprechend ausgestaltetes künstliches Kniegelenk 1 auch in einer Orthese oder einem Exoskelett eingesetzt werden. Statt eines Ersatzes eines natürlichen Gelenkes wird dann das jeweilige künstliche Kniegelenk medial und/oder lateral an dem natürlichen Gelenk angeordnet. Im dargestellten Ausführungsbeispiel ist das künstliche Kniegelenk 1 in Gestalt eines Prothesenkniegelenkes mit einem Oberteil 10 mit einer anterioren oder in Gehrichtung gelegenen oder vorderen Seite 11 und einer posterioren Seite 12, die der anterioren Seite 11 gegenüberliegt, ausgebildet. An dem Oberteil 10 ist ein Unterteil 20 schwenkbar um eine Schwenkachse 15 gelagert. Auch das Unterteil 20 weist eine anteriore Seite 21 oder vordere Seite und eine posteriore Seite 22 oder hintere Seite auf. In dem dargestellten Ausführungsbeispiel ist das Kniegelenk 1 als ein monozentrisches Kniegelenk ausgebildet, grundsätzlich ist es auch möglich, ein polyzentrisches Kniegelenk entsprechend zu steuern. An dem distalen Ende des Unterteils 20 ist ein Fußteil 30 angeordnet, das entweder als ein starres Fußteil 30 mit einem unbeweglichen Fußgelenk oder aber mit einer Schwenkachse 35 mit dem Unterteil verbunden sein kann, um einen an den natürlichen Bewegungsablauf angenäherten Bewegungsablauf zu ermöglichen.

Zwischen der posterioren Seite 12 des Oberteils 10 und der posterioren Seite 22 des Unterteils 20 wird der Kniewinkel KA gemessen. Der Kniewinkel KA kann über einen Kniewinkelsensor 25, der im Bereich der Schwenkachse 15 angeordnet sein kann, direkt gemessen werden. Der Kniewinkelsensor 25 kann mit einem Momentensensor gekoppelt sein oder einen solchen aufweisen, um eine Kniemoment um die Gelenkachse 15 zu erfassen. An dem Oberteil 10 ist ein Inertialwinkelsensor oder eine IMU 51 angeordnet, der die Raumlage des Oberteils 10 misst, beispielsweise im Verhältnis zu einer konstanten Kraftrichtung, beispielsweise der Gravitationskraft G, die vertikal nach unten zeigt. An dem Unterteil 20 ist ebenfalls ein Inertialwinkelsensor oder eine IMU 53 angeordnet, um die Raumlage des Unterteils während der Benutzung des Prothesenbeines zu ermitteln.

Zusätzlich zu dem Inertialwinkelsensor 53 kann an dem Unterteil 20 oder dem Fußteil 30 ein Beschleunigungssensor und/oder Querkraftsensor 53 angeordnet sein. Über einen Kraftsensor oder Momentensensor 54 an dem Unterteil 20 oder Fußteil 30 kann eine auf das Unterteil 20 wirkende Axialkraft FA oder ein um die Knöchelgelenksachse 35 wirkendes Knöchelmoment ermittelt werden kann.

Zwischen dem Oberteil 10 und dem Unterteil 20 ist eine Widerstandseinrichtung 40 angeordnet, um eine Verschwenkbewegung des Unterteils 20 relativ zu dem Oberteil 10 zu beeinflussen. Die Widerstandseinrichtung 40 kann als passiver Dämpfer, als Antrieb oder als ein sogenannter semiaktiver Aktuator ausgebildet sein, mit dem es möglich ist, Bewegungsenergie zu speichern und zu einem späteren Zeitpunkt gezielt wieder abzugeben, um Bewegungen abzubremsen oder zu unterstützen. Die Widerstandseinrichtung 40 kann als lineare oder rotatorische Widerstandseinrichtung ausgebildet sein. Die Widerstandseinrichtung 40 ist mit einer Steuerungseinrichtung 60 verbunden, beispielsweise verkabelt oder über eine drahtlose Verbindung, die wiederum mit zumindest einem der Sensoren 25, 51, 52, 53, 54 gekoppelt ist. Die Steuerungseinrichtung 60 verarbeitet die von den Sensoren übermittelten Signale elektronisch mit Prozessoren, Recheneinheiten oder Computern. Sie weist eine elektrische Energieversorgung sowie zumindest eine Speichereinheit auf, in der Programme und Daten gespeichert sind und in der ein Arbeitsspeicher zur Verarbeitung von Daten bereit steht. Nach der Verarbeitung der Sensordaten erfolgt die Ausgabe eines Aktivierungs- oder Deaktivierungsbefehles, mit dem die Widerstandseinrichtung 40 aktiviert oder deaktiviert wird. Durch Aktivieren eines Aktuators in der Widerstandseinrichtung 40 kann beispielsweise ein Ventil geöffnet oder geschlossen oder ein Magnetfeld erzeugt werden, um ein Dämpfungsverhalten zu verändern.

An dem Oberteil 10 des Prothesenkniegelenkes 1 ist ein Prothesenschaft befestigt, der zur Aufnahme eines Oberschenkelstumpfes dient. Über den Oberschenkelstumpf ist das Prothesenbein mit dem Hüftgelenk 16 verbunden, auf der anterioren Seite des Oberteils 10 wird ein Hüftwinkel HA gemessen, der zwischen einer vertikalen Linie durch das Hüftgelenk 16 und der Längserstreckung des Oberteils 10 und der Verbindungslinie zwischen dem Hüftgelenk 16 und der Kniegelenksachse 15 auf der anterioren Seite 11 angetragen ist. Wird der Oberschenkelstumpf angehoben und das Hüftgelenk 16 flektiert, verringert sich der Hüftwinkel HA, beispielsweise beim Hinsetzen. Umgekehrt vergrößert sich der Hüftwinkel HA bei einer Extension, beispielsweise beim Aufstehen oder ähnlichen Bewegungsabläufen, zum Beispiel beim Heruntertreten einer Pedale beim Fahrradfahren.

In der Figur 2 ist schematisch das Prothesenbein mit dem künstlichen Kniegelenk zwischen dem Oberteil 10 und dem Unterteil 20 dargestellt. Der Prothesenfuß 30 ist auf einer Pedale 2 aufgesetzt und führt eine Kreisbewegung entlang des Pedalweges aus. In der dargestellten Position befindet sich das Unterteil 20, bzw. die Längserstreckung des Unterteils 20, in einer senkrechten Stellung, die Neigung des Unterteils 20 zu der Senkrechten G beträgt somit 0°, der Rollwinkel ist somit 0. Der Flexionswinkel α_{K}, der sich aus der Verschwenkung des Oberteils 10 relativ zu dem Unterteil 20 um die Knieachse ergibt, ist die Änderung zu dem vollständig extendierten oder gestreckten Prothesenbein. Der Flexionswinkel α_{K} errechnet sich aus der Differenz zwischen 180° und dem Kniewinkel KA. Beim Ausführen einer Pedalierbewegung, wie sie beim Fahrradfahren zum Antreiben des Fahrrades notwendig ist, werden zyklische Bewegungen ausführt. Kräfte von dem Fußteil 30 auf die Pedale 20 werden in dem Krafteinleitungspunkt PF, der in der Figur 1 dargestellt ist, eingeleitet. In der Regel sind dies Druckkräfte, da ein Prothesennutzer oder ein Orthesennutzer mit eingeschränkten motorischen Fähigkeiten in der Muskulatur nur schwer in der Lage ist, Kräfte aufzubringen, die entgegen der Gravitationsrichtung wirken. Aufgrund der fehlenden Muskelanbindungen an das Unterteil 20 sind in der Regel keine Kräfte auf die Pedale 2 aufbringbar, die in Horizontalrichtung wirken. Da aus Sicherheitsgründen das Fußteil 30 nicht auf der Pedale 2 fixiert ist, werden auch keine entgegen der Schwerkraftrichtung wirkenden Kräfte, beispielsweise durch Aktivierung des Hüftbeugemuskels, übertragen. Bei der Pedalierbewegung sind daher Flexionswiderstände und auch Extensionswiderstände unerwünscht, da diese einer Drehbewegung der Pedale 2 um die Drehachse der Tretkurbel entgegenstehen.

Daher ist es vorgesehen, dass zumindest der Widerstand, insbesondere Flexionswiderstand und gegebenenfalls auch ein Extensionswiderstand, in Abhängigkeit von der Erkennung einer zyklischen Bewegung beim Fahrradfahren reduziert wird. Diese Erkennung und Reduzierung des W ist möglichst schnell auszuführen, ohne dass der Nutzer des künstlichen Kniegelenkes außer der Ausführung der Fahrradfahrbewegung weitere Maßnahmen ergreifen muss. Ist der zyklische Bewegungsablauf, der mit dem Fahrradfahren einhergeht, einmal erkannt, bleibt der Fahrradfahrmodus eingestellt, bis eine Änderungen von Parametern oder Sensorwerten erkannt wurde, die auf eine Beendigung des Fahrradfahrens hindeutet, was nicht unbedingt einhergehen muss mit einer Beendigung der zyklischen Bewegung.

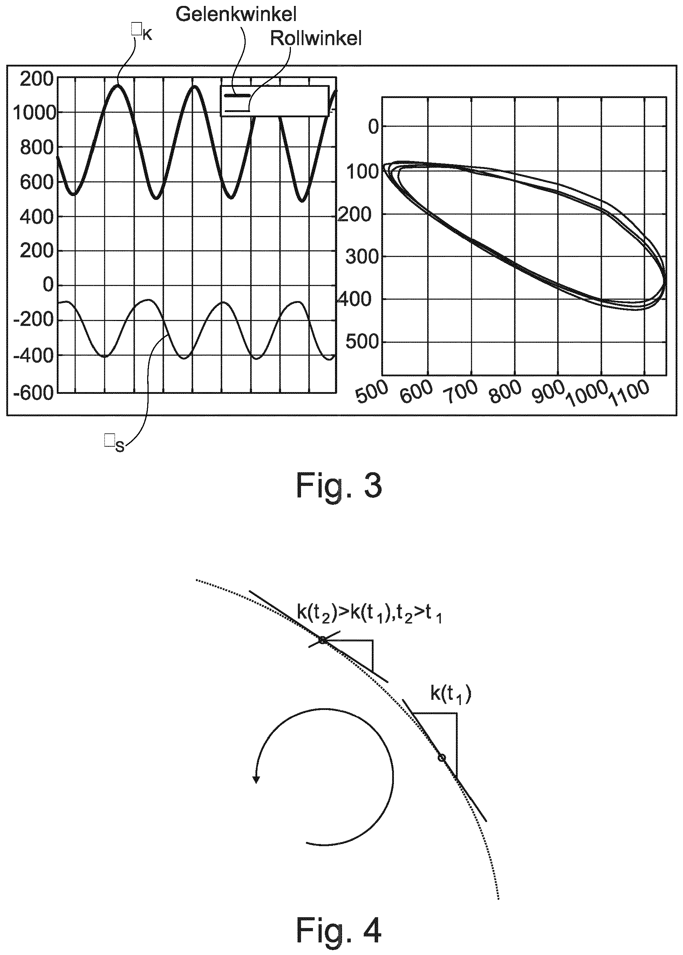

In der Figur 3 ist der Flexionswinkel α_{K} und der Rollwinkel α_{S} als Neigungswinkel des Unterteils 20 im Raum dargestellt. Der linken Darstellung ist zu entnehmen, dass beide Winkel im Wesentlichen zyklisch während des Fahrradfahrens verlaufen und bei einer gleichmäßigen Drehzahl der Pedale eine gleichförmige Veränderung durchlaufen. Die rechte Darstellung ist die Darstellung beider Werte in einem X-Y-Diagramm. Durch die Pedalbewegung ergibt sich eine geschlossene, konvexe, zweidimensionale Kurve, wobei der Flexionswinkel α_{K} auf der X-Achse und der Unterschenkelwinkel α_{S} auf der Y-Achse aufgetragen ist. Sofern eine gleichförmige und zyklische Bewegung erkannt wurde, und zwar über einen festgelegten Zeitraum, beispielsweise über zwei Pedalumdrehungen, wird der Widerstand der Widerstandseinrichtung verringert. Beim Fahrradfahren wird die Kurve in der rechten Darstellung der Figur 3 gegen den Uhrzeigersinn durchlaufen, wobei der zeitliche Verlauf der Tangentensteigung der Kurve, wie er in der Figur 4 dargestellt ist, nach rechts gelesen wird und daher monoton steigend ist. Die Steigung k₁ zu einem bestimmten Zeitpunkt t₁ wird aus dem Quotienten der Horizontalkomponente und Vertikalkomponente errechnet, die Steigung k₂ der Tangente zu einem späteren Zeitpunkt t₂ ist im dargestellten Ausführungsbeispiel größer. Die Tangentensteigung ist der Quotient der Änderungen der X- und Y-Komponente, also der Quotient der Winkelgeschwindigkeiten des Flexionswinkels α_{K} und des Neigungswinkels α_{S} des Unterteils 20.

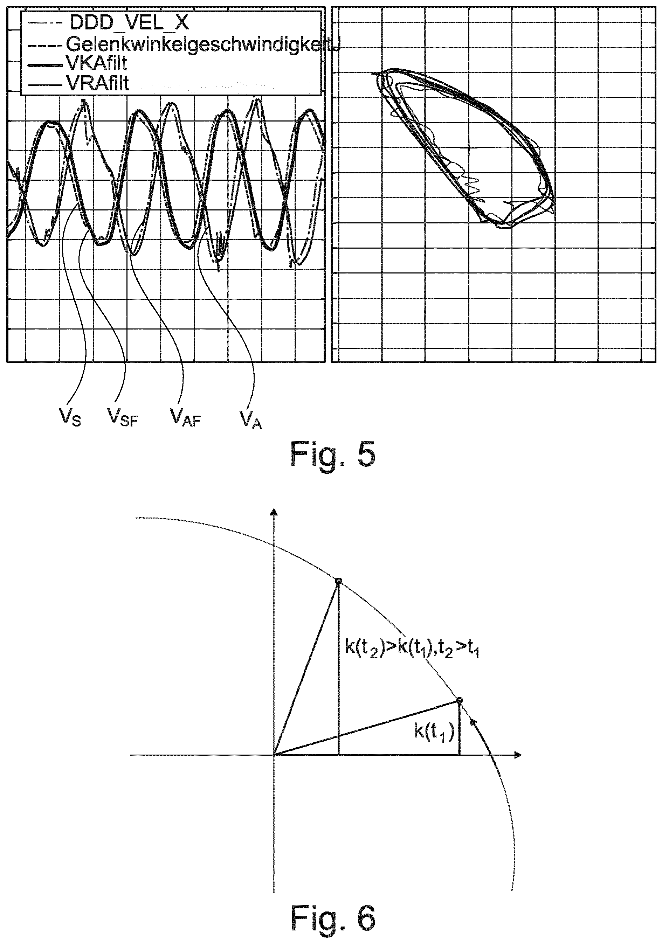

Eine Variante der Berechnung oder Ermittlung der Tangentensteigungen ist in den Figuren 5 und 6 dargestellt. In der Figur 5 sind die Winkelgeschwindigkeiten sowohl der Messungen als auch gefilterter Messungen dargestellt. Die Winkelgeschwindigkeit Vs des Unterteils und die Winkelgeschwindigkeit V_{A} des Flexionswinkels weisen beispielsweise aufgrund Erschütterungen oder Messfehlern Störsignale auf, die durch die ungleichmäßigen Kurvenverläufe kenntlich gemacht sind. Die gefilterten Winkelgeschwindigkeitssignale V_{SF} und V_{AF} sind ebenfalls aufgetragen. In der rechten Darstellung sind die Werte im X-Y-Diagramm mit Koordinatenursprung am Kreuz dargestellt. Auch hier sind die Unterschiede zwischen den vergleichsweise glatten Kurvenverläufen und den unruhigen Kurvenverläufen deutlich. Über die Vorschrift gemäß der Figur 4 wird die Steigung der Verbindungslinie zwischen dem Ursprung X und dem jeweiligen Punkt auf der Kurve im X-Y-Diagramm zum Zeitpunkt t₁ und Zeitpunkt t₂ errechnet. Bei einer Kniewinkelgeschwindigkeit V_{A}= 0 würde ein Vorzeichensprung von plus unendlich auf minus unendlich stattfinden. Ein solcher Wert wird bei der Betrachtung ausgenommen, indem die Auswertung nur bei Überschreiten eines Grenzwertes für die Flexionswinkelgeschwindigkeit stattfindet. Grundsätzlich können auch kleinere Abweichungen der Monotoniebedingung, wie oben erläutert wurde, zulässig sein, um das Gleichförmigkeitskriterium weiterhin als erfüllt anzusehen.

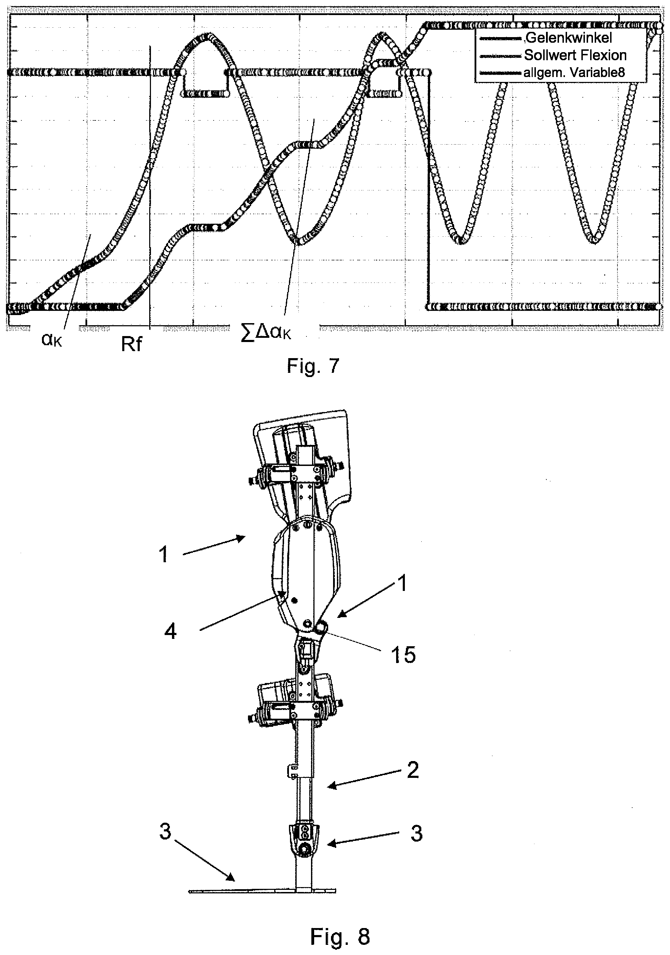

Um zu erkennen, ob die zyklische Bewegung des Fahrradfahrens ausgeführt wird, wird der Betrag der Flexionswinkeländerungen ΣΔα_{K}aufsummiert dabei der Flexionswinkel und der Rollwinkel und/oder die Orientierung des Oberteils im Raum betrachtet. In dem Diagramm der Figur 7 ist der Verlauf des Flexionswinkels α_{K} zum Beginn des Fahrradfahrens aufgezeichnet. Von einem gestreckten Bein ausgehend wird eine Flexion mit anschließender Extension und wieder Flexion und Extension durchgeführt. Der Widerstand Rf bleibt zunächst auf einem hohen Niveau. Der Zählwert *Σ* Δα*_{K}* wird immer dann erhöht, wenn eine ausreichende absolute Kniewinkelgeschwindigkeit, also eine ausreichend hohe Änderung des Flexionswinkels α_{K} vorliegt. Im Bereich der Bewegungsumkehr mit einer verringerten Flexionswinkeländerung wird der Zählwert nicht fortgeschrieben, um die ausreichende Sicherheit bei der Erkennung der Fahrradfahrbewegung nicht zu gefährden. Gleiches gilt bei dem Durchlauf nach dem Berechnen einer maximalen Extension, also einem unteren relativen Minimum des Flexionswinkels α_{K}. Erreicht der Zähler den festgelegten Grenzwert, beispielsweise einen Betrag der Flexionswinkeländerung von 240°, was an dem waagerechten Verlauf des Zählers Σ *Δα_{K}* zu erkennen ist, wird der Widerstand Rf auf den gewünschten Wert reduziert, beispielsweise auf nahezu 0 abgesetzt. Eine weitere Fortschreibung des Zählwertes findet nicht statt, da das Gleichförmigkeitskriterium ausreichend erfüllt ist.

In der Figur 8 ist in einer schematischen Darstellung ein Ausführungsbeispiel einer Orthese mit einem Oberteil 10 und einem schwenkbar um eine Schwenkachse 15 daran gelagerten Unterteil 20 gezeigt, mit der das Verfahren ebenfalls ausgeführt werden kann. Zwischen dem Oberteil 10 und dem Unterteil 20 ist dadurch ein künstliches Kniegelenk 1 ausgebildet, das in dem dargestellten Ausführungsbeispiel lateral zu einem natürlichen Kniegelenk angeordnet ist. Neben einer einseitigen Anordnung von Oberteil 10 und Unterteil 20 relativ zu einem Bein können auch zwei Oberteile und Unterteile medial und lateral zu einem natürlichen Bein angeordnet sein. Das Unterteil 20 weist an seinem distalen Ende ein Fußteil 30 auf, das um eine Knöchelgelenksachse 35 schwenkbar zu dem Unterteil 20 gelagert ist. Das Fußteil 30 weist eine Fußplatte auf, auf der ein Fuß oder Schuh aufgesetzt werden kann. Sowohl an dem Unterteil 20 als auch an dem Oberteil 30 sind Befestigungseinrichtungen zum Festlegen an dem Unterschenkel bzw. Oberschenkel angeordnet. An dem Fußteil 30 können auch Einrichtungen zum Festlegen des Fußes auf dem Fußteil 30 angeordnet sein. Die Befestigungseinrichtungen können als Schnallen, Gurte, Spangen oder dergleichen ausgebildet sein, um die Orthese lösbar an dem Bein des Nutzers anlegen und zerstörungsfrei wieder abnehmen zu können. An dem Oberteil 10 ist die Widerstandseinrichtung 40 befestigt, die sich an dem Unterteil 20 und an dem Oberteil 10 abstützt und einen einstellbaren Widerstand gegen eine Verschwenkung um die Schwenkachse 15 bereitstellt. Die Sensoren und die Steuerungseinrichtung, die weiter oben im Zusammenhang mit dem Ausführungsbeispiel der Prothese beschrieben wurden, sind entsprechend auch an der Orthese vorhanden.

## Patentansprüche

1. Verfahren zur Steuerung einer Prothese oder Orthese der unteren Extremität mit einem Oberteil (10) und einem mit dem Oberteil (10) über ein Kniegelenk verbundenes Unterteil (20), das um eine Gelenkachse (15) relativ zu dem Oberteil (10) verschwenkbar gelagert ist, wobei zwischen dem Oberteil (10) und dem Unterteil (20) eine verstellbare Widerstandseinrichtung (40) angeordnet ist, über die auf der Grundlage von Sensordaten ein Widerstand (Rf) verändert wird, wobei Zustandsinformationen über Sensoren erfasst, eine vom Gehen abweichende zyklische Bewegung ermittelt und der Widerstand (Rf) während der zyklischen Bewegung auf ein niedriges Niveau eingestellt wird, wobei die Ermittlung der zyklischen Bewegung folgende Schritte aufweist:
a. Erfassung des Flexionswinkels (α_{K}) und zumindest eines Absolutwinkels (α_{S}) des Unterteils (20) und/oder des Oberteils (10) über zumindest einen Bewegungszyklus
b. Erkennung der zyklischen Bewegung aus der Relativbewegung von Oberteil (10) und Unterteil (20) und der Absolutbewegungen von Oberteil (10) und/oder Unterteil (20) im Raum, **dadurch gekennzeichnet, dass** zur Ermittlung der zyklischen Bewegung der Phasenraum zweier Winkel-Kenngrößen und/oder deren Ableitungen herangezogen wird, insbesondere die Drehrichtung im Phasenraum bestimmt und herangezogen wird.

2. Verfahren nach Anspruch 1, **dadurch gekennzeichnet, dass** die Reduzierung des Widerstandes (Rf) nur dann erfolgt, wenn eine Flexionswinkeländerung (∑Δα_{K} ), insbesondere der Betrag der Flexionswinkeländerung, aufsummiert über ein Intervall, in dem bestimmte Bedingungen erfüllt sind, größer als ein festgelegter Grenzwert, insbesondere größer als 240° ist

3. Verfahren nach Anspruch 1 oder 2, **dadurch gekennzeichnet, dass** eine zyklische Bewegung nur dann festgestellt wird, wenn der Flexionswinkel (α_{K}) größer als ein Grenzwert, insbesondere größer als10°, insbesondere größer als 15° ist.

4. Verfahren nach einem der der voranstehenden Ansprüche, **dadurch gekennzeichnet, dass** eine zyklische Bewegung nur dann festgestellt wird, wenn der Neigungswinkel (α_{S}) des Unterteils (20) und/oder der Flexionswinkel (α_{K}) bestimmte Grenzwerte nicht über- und/oder unterschreiten.

5. Verfahren nach einem der voranstehenden Ansprüche, **dadurch gekennzeichnet, dass** eine zyklische Bewegung nur dann festgestellt wird, wenn der Neigungswinkel (α_{S}) des Unterteils (20) nach vorn relativ zu der Senkrechten (G) kleiner als ein Grenzwert, insbesondere kleiner als 5° ist.

6. Verfahren nach einem der voranstehenden Ansprüche, **dadurch gekennzeichnet, dass** die Beendigung der zyklischen Bewegung dadurch erkannt wird, dass das Unterteil (20) für einen vorgegebenen Zeitraum von einer Axialkraft (FA) entlastet wird und die Axialkraft (FA) unter einen vorgegebenen Grenzwert sinkt und der Flexionswinkel (α_{K}) einen Grenzwert unterschreitet oder die Neigung (α_{S}) des Unterteils (20) zur Senkrechten (G) einen Grenzwert überschreitet.

7. Verfahren nach einem der voranstehenden Ansprüche, **dadurch gekennzeichnet, dass** bei Unterbrechung der zyklischen Bewegung bei gleichzeitiger Beaufschlagung des Unterteils (20) mit einer Axialkraft (FA) eine Extension des Kniegelenkes (1) unterhalb eines festgelegten Flexionswinkels (α_{K}) verhindert und der Flexionswiderstand (Rf) erhöht wird und nach Erreichen eines Endanschlages, nachfolgender Flexion und erneuter Extension die Widerstandseinstellung für die zyklische Bewegung erneut eingestellt wird.

8. Verfahren nach einem der voranstehenden Ansprüche, **dadurch gekennzeichnet, dass** zur Ermittlung der zyklischen Bewegung die Winkelgeschwindigkeiten des Flexionswinkels (αₖ) und des Neigungswinkels (α_{S}) errechnet und der Quotient der Winkelgeschwindigkeiten in festgelegten Zeitabschnitten ermittelt wird.

9. Verfahren nach Anspruch 8, **dadurch gekennzeichnet, dass** Änderungen des Quotienten der Winkelgeschwindigkeiten als Tangentensteigungen in festgelegten Zeitabschnitten ermittelt werden.

10. Verfahren nach Anspruch 8 oder 9, **dadurch gekennzeichnet, dass** eine Feststellung der zyklischen Bewegung erfolgt, wenn der zeitliche Verlauf der Tangentensteigungen monoton steigend ist.

11. Verfahren nach einem der Ansprüche 8 bis 10, **dadurch gekennzeichnet, dass** eine Auswertung der Winkelgeschwindigkeiten nur bei Überschreiten eines Grenzwertes der Flexionswinkelgeschwindigkeit erfolgt.

12. Verfahren nach einem der voranstehenden Ansprüche, **dadurch gekennzeichnet, dass** aus dem Kniewinkel (KA) und/oder Absolutwinkeln von Oberteil (10) und Unterteil (20) die Bahnkurve des Fußteils (30) und/oder deren Ableitungen relativ zu einem ermittelten Hüftdrehpunkt bestimmt wird und für die Ermittlung der zyklischen Bewegung herangezogen wird.

## Claims

1. A method for controlling a prosthesis or orthosis of the lower extremity, the prosthesis or orthosis having an upper part (10) and a lower part (20) which is connected to the upper part (10) via a knee joint and is mounted so as to be pivotable relative to the upper part (10) about a joint axis (15), wherein an adjustable resistance device (40) is arranged between the upper part (10) and the lower part (20), by means of which resistance device (40) a resistance (Rf) is modified on the basis of sensor data, wherein state information is detected via sensors, a cyclical movement different than walking is determined, and the resistance (Rf) is adjusted to a low level during the cyclical movement, wherein the determination of the cyclical movement comprises the following steps:
a. detecting the flexion angle (α_{K}) and at least one absolute angle (α_{S}) of the lower part (20) and/or of the upper part (10) over at least one movement cycle,
b. identifying the cyclical movement from the relative movement of upper part (10) and lower part (20) and from the absolute movements of upper part (10) and/or lower part (20) in space, **characterized in that** the phase space of two angle parameters and/or their derivatives is used to determine the cyclical movement, in particular the direction of rotation in the phase space is determined and used.

2. The method as claimed in claim 1, **characterized in that** the reduction of the resistance (Rf) takes place only when a flexion angle change (ΣΔα_{K}), in particular the amount of the flexion angle change, added up over an interval in which certain conditions are met, is greater than a specified limit value, in particular greater than 240°.

3. The method as claimed in claim 1 or 2, **characterized in that** a cyclical movement is ascertained only if the flexion angle (α_{K}) is greater than a limit value, in particular greater than 10°, in particular greater than 15°.

4. The method as claimed in one of the preceding claims, **characterized in that** a cyclical movement is ascertained only if the angle of inclination (αₛ) of the lower part (20) and/or the flexion angle (α_{K}) does not exceed and/or fall below certain limit values.

5. The method as claimed in one of the preceding claims, **characterized in that** a cyclical movement is ascertained only if the angle of inclination (αₛ) of the lower part (20) forward relative to the vertical (G) is less than a limit value, in particular less than 5°.

6. The method as claimed in one of the preceding claims, **characterized in that** the end of the cyclical movement is identified from the fact that the lower part (20) is relieved of an axial force (FA) for a predetermined period of time and the axial force (FA) falls below a predetermined limit value and the flexion angle (α_{K}) falls below a limit value or the inclination (αₛ) of the lower part (20) to the vertical (G) exceeds a limit value.

7. The method as claimed in one of the preceding claims, **characterized in that**, when the cyclical movement is interrupted while the lower part (20) is simultaneously subjected to an axial force (FA), an extension of the knee joint (1) below a specified flexion angle (α_{K}) is prevented and the flexion resistance (Rf) increased and, after an end stop is reached, followed by flexion and renewed extension, the resistance is set anew for the cyclical movement.

8. The method as claimed in one of the preceding claims, **characterized in that**, in order to determine the cyclical movement, the angular velocities of the flexion angle (α_{K}) and of the angle of inclination (αₛ) are calculated, and the quotient of the angular velocities is determined in specified time segments.

9. The method as claimed in claim 8, **characterized in that** changes in the quotient of the angular velocities are determined as tangent slopes in specified time segments.

10. The method as claimed in claim 8 or 9, **characterized in that** the cyclical movement is ascertained when the time profile of the tangent slopes is increasing monotonically.

11. The method as claimed in one of claims 8 to 10, **characterized in that** an evaluation of the angular velocities takes place only when a limit value of the flexion angle velocity is exceeded.

12. The method as claimed in one of the preceding claims, **characterized in that** the trajectory of the foot part (30) and/or its derivatives relative to a determined hip rotation point is determined from the knee angle (KA) and/or absolute angles of upper part (10) and lower part (20) and is used for the determination of the cyclical movement.

## Revendications

1. Procédé de commande d'une prothèse ou d'une orthèse du membre inférieur, comprenant une partie supérieure (10) et une partie inférieure (20) qui est reliée à la partie supérieure (10) par l'intermédiaire d'une articulation de genou et est montée pivotante autour d'un axe d'articulation (15) par rapport à la partie supérieure (10), un dispositif de résistance réglable (40) étant disposé entre la partie supérieure (10) et la partie inférieure (20), lequel permet de modifier une résistance (Rf) sur la base des données de capteurs, dans lequel des informations d'état sont acquises par l'intermédiaire de capteurs, un mouvement cyclique qui diffère de la marche est déterminé, et la résistance (Rf) est réglée à un niveau bas pendant le mouvement cyclique, la détermination du mouvement cyclique comprenant les étapes suivantes consistant à :
a. détecter l'angle de flexion (α_{K}) et au moins un angle absolu (α_{S}) de la partie inférieure (20) et/ou de la partie supérieure (10) sur au moins un cycle de mouvement,
b. reconnaître le mouvement cyclique à partir du mouvement relatif de la partie supérieure (10) et de la partie inférieure (20) et des mouvements absolus de la partie supérieure (10) et/ou de la partie inférieure (20) dans l'espace,
**caractérisé en ce que** pour déterminer le mouvement cyclique, on prend en compte l'espace des phases de deux paramètres angulaires et/ou leurs dérivées, en particulier on détermine et prend en compte le sens de rotation dans l'espace des phases.

2. Procédé selon la revendication 1,
**caractérisé en ce que** la résistance (Rf) n'est réduite que si une modification de l'angle de flexion (ΣΔα_{K}), en particulier la valeur absolue de la modification de l'angle de flexion, totalisée pendant un intervalle où certaines conditions sont satisfaites, est supérieure à une valeur limite définie, en particulier supérieure à 240°.

3. Procédé selon la revendication 1 ou 2,
**caractérisé en ce qu'**un mouvement cyclique n'est déterminé que si l'angle de flexion (α_{K}) est supérieur à une valeur limite, en particulier supérieur à 10°, en particulier supérieur à 15°.

4. Procédé selon l'une des revendications précédentes,
**caractérisé en ce qu'**un mouvement cyclique n'est déterminé que si l'angle d'inclinaison (α_{S}) de la partie inférieure (20) et/ou l'angle de flexion (α_{K}) ne passent pas au-dessus et/ou en dessous de certaines valeurs limites.

5. Procédé selon l'une des revendications précédentes,
**caractérisé en ce qu'**un mouvement cyclique n'est déterminé que si l'angle d'inclinaison (α_{S}) de la partie inférieure (20) vers l'avant par rapport à la verticale (G) est inférieur à une valeur limite, en particulier inférieur à 5°.

6. Procédé selon l'une des revendications précédentes,
**caractérisé en ce que** la fin du mouvement cyclique est reconnue par le fait que la partie inférieure (20) est déchargée d'une force axiale (FA) pendant une durée prédéfinie et que la force axiale (FA) descend en dessous d'une valeur limite prédéfinie et que l'angle de flexion (α_{K}) passe en dessous d'une valeur limite ou que l'inclinaison (α_{S}) de la partie inférieure (20) par rapport à la verticale (G) passe au-dessus d'une valeur limite.

7. Procédé selon l'une des revendications précédentes,
**caractérisé en ce que**, en cas d'interruption du mouvement cyclique, accompagnée d'une sollicitation simultanée de la partie inférieure (20) par une force axiale (FA), une extension de l'articulation de genou (1) en dessous d'un angle de flexion défini (α_{K}) est empêchée et la résistance à la flexion (Rf) est augmentée, et après avoir atteint une butée de fin de course, suivie d'une flexion et d'une nouvelle extension, le réglage de la résistance du mouvement cyclique est réajusté.

8. Procédé selon l'une des revendications précédentes,
**caractérisé en ce que**, pour déterminer le mouvement cyclique, les vitesses angulaires de l'angle de flexion (α_{K}) et de l'angle d'inclinaison (α_{S}) sont calculées, et le quotient des vitesses angulaires est déterminé à des intervalles de temps définis.

9. Procédé selon la revendication 8,
**caractérisé en ce que** les modifications du quotient des vitesses angulaires sont déterminées sous forme de gradients tangentiels à des intervalles de temps définis.

10. Procédé selon la revendication 8 ou 9,
**caractérisé en ce que** le mouvement cyclique est déterminé si la progression temporelle des gradients tangentiels est croissante monotone.

11. Procédé selon l'une des revendications 8 à 10,
**caractérisé en ce que** les vitesses angulaires ne sont évaluées que si la vitesse angulaire de flexion passe au-dessus d'une valeur limite.

12. Procédé selon l'une des revendications précédentes,
**caractérisé en ce que** la trajectoire de la partie pied (30) et/ou ses dérivées par rapport à un point de pivot de hanche déterminé sont définies à partir de l'angle de genou (KA) et/ou des angles absolus de la partie supérieure (10) et de la partie inférieure (20) et sont prises en compte pour déterminer le mouvement cyclique.
